# EUROPEAN PATENT SPECIFICATION

(11) **EP 1 767 947 B1**
(45) Date of publication and mention of the grant of the patent: **29.10.2008**
(21) Application number: 06019913.0
(22) Date of filing: 22.09.2006
(51) Int. Cl.: G01N 33/84

(54) **An immunoassay method and kit to leucomalachite green and malachite green**
Immunoassay-Verfahren und Kit für Leukomalachitgrün und Malachitgrün
Procédé de dosage immunologique et trousse pour vert leucomalachite et vert malachite

(30) Priority: 22.09.2005 EP 05077175
(43) Date of publication of application: 28.03.2007
(73) Proprietor: Randox Laboratories Ltd., Ardmore, Diamond Road Crumlin, Co. Antrim Northern Ireland, BT29 4QY (GB)
(72) Inventor: Benchikh, El Ouard, Crumlin BT29 4QY, Northern Ireland (GB); McConnell, Robert Ivan, Crumlin BT29 4QY, Northern Ireland (GB); Fitzgerald, Stephen Peter, Crumlin BT29 4QY, Northern Ireland (GB); Lowry, Andrew Philip, Crumlin BT29 4QY, Northern Ireland (GB)
(74) Representative: O'Connell, Maura

(56) References cited:
- EP-A- 1 312 923
- FR-A- 936 371
- US-A- 3 310 401
- US-A- 3 739 000
- US-A- 5 605 616
- US-A1- 2003 065 150
- US-B1- 6 689 616
- KOSTURKO L D ET AL: "SELECTIVE REPRESSION OF TRANSCRIPTION BY BASE SEQUENCE SPECIFIC SYNTHETIC POLYMERS" BIOCHEMISTRY, vol. 18, no. 26, 1979, pages 5751-5756, XP002360688
- RITCHIE C D ET AL: "Application of Linear Free Energy Relationships to Some Reactions of Triarylmethane Derivatives" JOURNAL OF THE AMERICAN CHEMICAL SOCIETY, vol. 84, no. 12, 1962, pages 2349-2356, XP002360689
- VALLE L ET AL: "Determination of the sum of malachite green and leucomalachite green in salmon muscle by liquid chromatography-atmospheric pressure chemical ionisation-mass spectrometry" JOURNAL OF CHROMATOGRAPHY, vol. 1067, no. 1-2, 4 March 2005 (2005-03-04), pages 101-105, XP004798972

## Description

### FIELD OF THE INVENTION

The present invention relates to novel immunogens, antibodies and conjugates, for use in immunoassays for the detection and / or determination of leucomalachite green and malachite green. The present invention also relates to an immunoassay method and kit for detecting or determining leucomalachite green and malachite green. By "detecting" is meant qualitatively analyzing for the presence or absence of leucomalachite green and/or malachite green in a sample. By "determining" is meant quantitatively analyzing for the amount of leucomalachite green and/or malachite green in a sample.

### BACKGROUND TO THE INVENTION

Malachite green is an ectoparasiticide, fungicide and antiseptic used in fish farming. However, it is potentially carcinogenic, mutagenic and teratogenic. Its illegal and public health threatening use in edible fish species, such as trout and eel, has been recognised since 1933. Following absorption, malachite green is rapidly reduced to its non-chromophorous metabolite, leucomalachite green, which is the prevalent residue detected during drug residue analysis of treated aquatic animals. Malachite green and leucomalachite green have the following structural formulae:

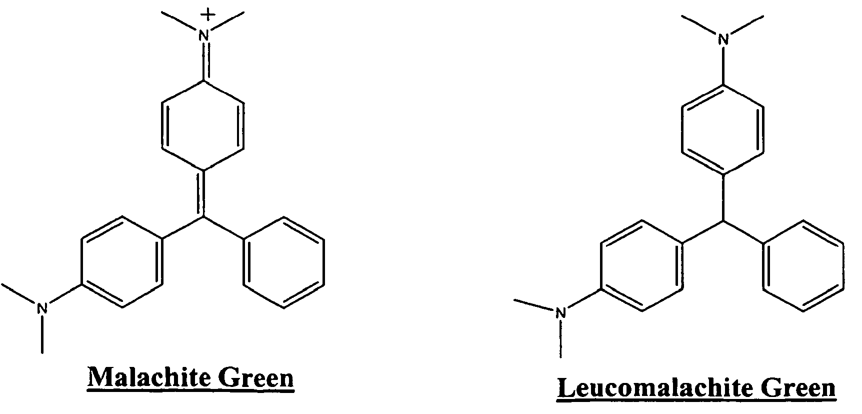

Malachite green and leucomalachite green recently acquired much attention from residue analysts, as they were found in cultured Atlantic salmon and hake (cf. Weekly EC Rapid Alert Reports for Food and Feed 2003/2004).

Methodology for the determination of malachite green and leucomalachite green is fairly limited. Malachite green has chromophores in the visible region of the spectrum (at 580-620nm), which is a considerable help in its analysis. Leucomalachite green, however, has a λmax at 260-270nm, making it difficult to analyze under the same conditions. A number of approaches have been taken to circumvent this problem. One method employs a post-column oxidation step, after HPLC separation and prior to detection, to oxidise leucomalachite green to malachite green, allowing detection at the higher wavelength. Commonly, lead oxide dispersed on Celite (Trade Mark), or on its own, packed into a short column has been used. In a variation on this, Klein et al. (E. Klein, M. Edelhaeuser and R. Lippold, Dtsch. Lebensm.-Rundsch., 1991, 87, 350) used lead dioxide to carry out a pre-column oxidation, measuring malachite green as the total of the parent and the leucometabolite. Electrochemical oxidation has been used as an alternative to lead dioxide. An alternative approach to the detection of both malachite green and leucomalachite green is to use LC-MS. Particle beam LC-MS has been used for confirmation of malachite green in catfish.

The European minimum required performance limit (MRPL), a quality parameter for residue laboratories, is set at 2µg/kg for the sum of malachite green and leucomalachite green. Recently, gas chromatography mass spectrometry (GC-MS) and other liquid chromatography tandem mass spectrometry (LC-MS/MS) methods were presented at the fifth edition of the Euro Residue Conference. Both GC-MS and LC-MS methods had favourable performances, securing the detection and identity of these residues at concentrations far below the settled MRPL.

Specific binding reactions, such as antibody-antigen interactions, have been used extensively in immunoassays to detect a variety of substances present in tissue extracts. Thus, for example, radioimmunoassays (RIAs) could be used for the determination of malachite green and leucomalachite green. Radioimmunoassays are very sensitive, but do require radionuclide tracers, for example ¹²⁵I and ³H. There are no known RIAs for malachite green and leucomalachite green.
Enzyme-linked immunosorbent assays (ELISAs) are a nonradioactive alternative that could be used for the qualitative and quantitative determination of malachite green and leucomalachite green. Recently, a test kit for malachite green has become commercially available. Bioo Scientific produce a malachite green ELISA Test Kit that is designed to detect malachite green only. This suggests that the Bioo Kit contains antibodies were raised to a malachite based immunogen, unlike the present invention which uses a leucomalachite based immunogen. This is further supported by information provided by Bioo Scientific which gives a cross-reactivity of 1 % towards leucomalachite green. As malachite green is rapidly and extensively metabolized to leucomalachite green, the Bioo Scientific immunoassay requires the incorporation of a sample pre-treatment step prior to antibody-antigen binding, to convert leucomalachite green to malachite green. This makes the immunoassay more time-consuming and more prone to experimental error through the use of additional steps in the assay, compared to the present invention.

### SUMMARY OF THE INVENTION

In a first aspect, the present invention provides an immunogen comprising leucomalachite green, coupled by way of a crosslinker, to an antigenicity-conferring carrier material, the crosslinker extending from the para, ortho or meta-position of the unsubstituted phenyl ring of leucomalachite green. The invention also provides antibodies raised against such immunogens. In addition, the invention concerns conjugates comprising leucomalachite green, covalently bonded by way of a crosslinker, to a detectable labelling agent, the crosslinker extending from the para, ortho or meta-position of the unsubstituted phenyl ring of leucomalachite green.

The immunogens and conjugates of the present invention can each be derived from haptens of the following formula I, in which the crosslinker (the crosslinker can comprise -X-Y-Z) extends from the para, ortho or meta-position of the unsubstituted phenyl ring of leucomalachite green: 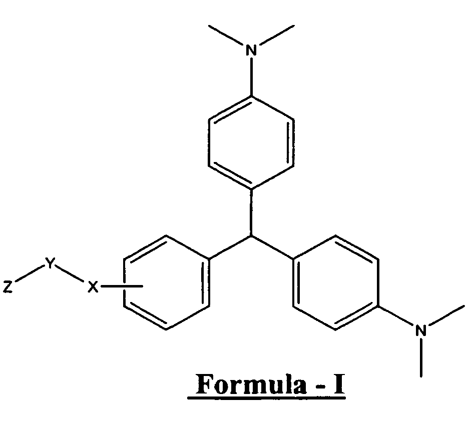 in which X is a first bivalent link, Y is a second bivalent link and Z is a reactive group conjugatable with an antigenicity-conferring carrier material (to produce an immunogen) or with a detectable labelling agent (to produce a conjugate).

Optionally, the immunogen comprises the structure of formula I comprising an uncoupled crosslinker prior to coupling to the antigenicity-conferring carrier material, wherein said uncoupled crosslinker comprises -X-Y-Z: 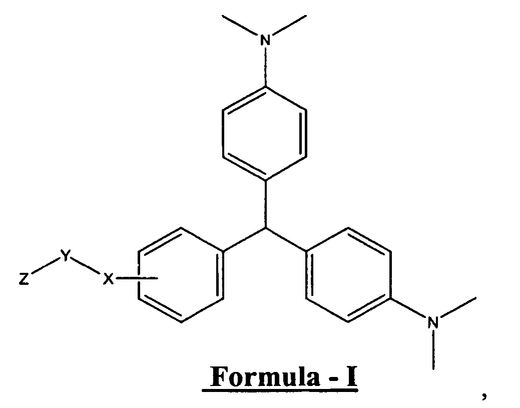 wherein X is a first bivalent link, Y is a second bivalent link and Z is a reactive group capable of coupling to the antigenicity-conferring carrier material.

Optionally, the conjugate comprises the structure of formula I comprising an uncoupled crosslinker prior to coupling to the detectable labelling agent, wherein said uncoupled crosslinker comprises -X-Y-Z: 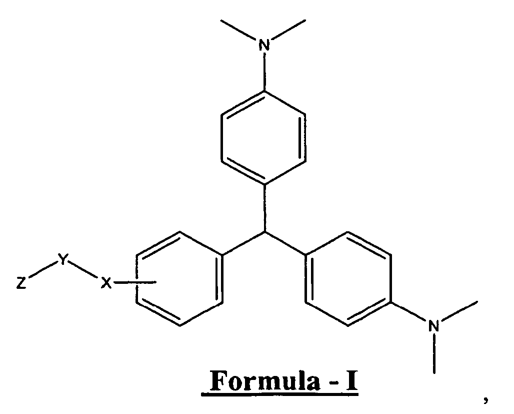 wherein X is a first bivalent link, Y is a second bivalent link and Z is a reactive group capable of coupling to the detectable labelling agent.

Preferably, for each of the immunogens and conjugates, the crosslinker extends from the para position of the unsubstituted phenyl ring of leucomalachite green.

Preferably, for each of the immunogens and conjugates, X is a heteroatom selected from O, S and N, most preferably X is an O atom for immunogens and X is most preferably an N atom for conjugates.

Preferably, for each of the immunogens and conjugates, Y is a C₁₋₁₀, more preferably a C₂₋₆, most preferably a C₃, substituted or unsubstituted straight chain, saturated alkylene moiety, or an arylene moiety. One suitable substituent is carbonyl (C=O).

Advantageously, Z (before conjugation with an antigenicity-conferring carrier material (to produce an immunogen) or with a detectable labelling agent (to produce a conjugate)) is selected from a carboxylic acid, a dithiopyridyl, a maleimide, an amino, a hydroxyl, a thiol, a thioester or an aldehyde moiety. Where Z, before conjugation to the antigenicity-conferring carrier material is a carboxylic acid (COOH), the oxygen of the hydroxyl group combines first with DCC and then NHS to form an ester with a powerful leaving group. Nucleophilic attack on the carbonyl group (C=O) of the ester functionality by a free amine group on the antigenicity-conferring carrier material results in an amide bond and formation of the desired immunogen. Where Z, before conjugation to the detectable labelling reagent, is a thiol group (in the case of a thioester, the thiol is formed through hydrolysis under basic conditions), a nucleophilic substitution reaction involving the thiol and BrCH₂C(O)NH-HRP results in bromine replacement by the thiol group and formation of the desired conjugate. The skilled reader is referred to Bioconjugate Techniques G. Hermanson, ed., Academic Press, 1996, 785 pp., whose contents are incorporated in its entirety, for details of the interaction between the Z reactive group and either the antigenicity-conferring carrier material or the detectable labelling agent, where Z is selected from the remainder of a carboxylic acid, a dithiopyridyl, a maleimide, an amino, a hydroxyl, a thiol, a thioester or an aldehyde moiety. Most advantageously, Z (before conjugation with an antigenicity-conferring carrier material (to produce an immunogen)) is a carboxy (COOH) moiety. Most advantageously, Z (before conjugation with a detectable labelling agent (to produce a conjugate)) is a thiol or a thioester moiety.

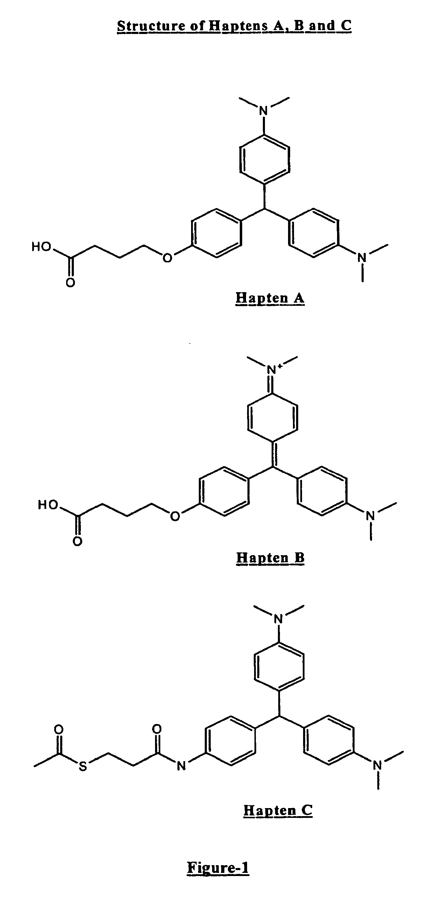

Most advantageously, the hapten is *p*-(3-carboxypropoxy) leucomalachite green (Hapten A). The structural formula of hapten A is presented in Figure 1. Preferably, the immunogen is *p*-(3-carboxypropoxy) leucomalachite green coupled to an antigenicity-conferring carrier material, optionally selected from bovine serum albumin (BSA) and bovine thyroglobulin (BTG).

Hapten derivatives of malachite green (and corresponding immunogens, antibodies and conjugates) can be prepared in a similar manner by derivatisation of malachite green with crosslinkers at the para, ortho or meta-positions, preferably the para position, of the unsubstituted phenyl ring. One such hapten derivative of malachite green is *p*-(3-carboxypropoxy) malachite green (hapten B), the structural formula of which is also illustrated in Figure 1.

The immunogens are prepared by coupling a hapten (as described above) to a modified or non-modified antigenicity-conferring carrier material. Preferably, the carrier material is a protein, a protein fragment, a synthetic polypeptide or a semisynthetic polypeptide. The immunogens obtained are then administered to mammalian hosts to elicit production of specific antibodies, optionally polyclonal antibodies, which are then used to develop immunoassays for leucomalachite green and malachite green, employing haptens conjugated to labelling agents as detection reagents.

In a still further aspect, the present invention concerns antibodies raised against the immunogens of the present invention, the antibodies being capable of binding with at least one structural epitope of leucomalachite green and malachite green.

Said antibody can bind at least one structural epitope of leucomalachite green and malachite green. In a still further aspect, the present invention concerns antibodies having specificity for leucomalachite green characterised by having cross-reactivity for malachite green. Said antibody can specifically bind leucomalachite green and comprise cross-reactivity for malachite green. Optionally, the antibodies (and methods and kits including those antibodies) have cross-reactivity of more than 10% for malachite green. Further optionally, the antibodies (and methods and kits including those antibodies) have cross-reactivity of more than 20% for malachite green. Still further optionally, the antibodies (and methods and kits including those antibodies) have cross-reactivity of more than 25% for malachite green. In each such case, the quoted optional minimum cross-reactivities for malachite green are with respect to 100% cross-reactivity for leucomalachite green.

The invention further provides a process of preparing the antibodies, the process comprising the steps of immunising an animal, preferably a vertebrate animal, most preferably a mammalian animal, by repeated administration of an immunogen of the present invention, and collecting the resulting serum from the immunised animal. Preferably, the process further comprises fixing said serum antibodies to a backing substrate, preferably a solid support, most preferably a polystyrene solid support. Preferably, the antibodies are polyclonal. Alternatively, the antibodies are monoclonal.

In a still further aspect, the present invention comprises a conjugate comprising the haptens disclosed herein covalently bonded to a detectable labelling agent. Preferably, the labelling agent is selected from an enzyme, a luminescent substance, a radioactive substance, or a mixture thereof. More preferably, the labelling agent is an enzyme, preferably a peroxidase, most preferably horseradish peroxidase (HRP). Alternatively, or additionally, the luminescent substance may be a bioluminescent, chemiluminescent or fluorescent material.

Preferably, the conjugate is *p*-(3-acetylthiopropanamido) leucomalachite covalently bonded to a detectable labelling agent. One such suitable labelling agent is horseradish peroxidase.

In a still further aspect, the present invention comprises a method for detecting or determining leucomalachite green and malachite green in a sample, the method comprising contacting the sample with at least one conjugate of the present invention, and with at least one antibody of the present invention; detecting or determining bound conjugate; and deducing from a calibration curve the presence of, or the amount of, leucomalachite green and malachite green, in the sample. This is a semi-quantitative method, whilst the following method is a quantitative method. The semi-quantitative method can, optionally, detect or determine an analyte selected from leucomalachite green, malachite green and mixtures of leucomalachite green and malachite green in a sample. Alternatively, the method comprises contacting a first fraction of the sample with at least one conjugate of the present invention, and with at least one antibody of the present invention and contacting another fraction of the sample with at least one antibody of the present invention; determining bound conjugate (to determine the combined amount of leucomalachite green and malachite green) in the first fraction and determining bound malachite green (to determine the amount of malachite green) in the second fraction; and deducing from calibration curves, the amount of each of leucomalachite green and malachite green in the sample. It will be understood that the determining step in the first fraction is a competitive immunoassay and the determination is arranged to determine the combined amount of leucomalachite green and malachite green. It will also be understood that the determining step in the second fraction is an immunoassay and the determination is arranged to determine the amount of bound malachite green. The latter determination step can be conducted using an appropriate spectroscopic method. The deducing step in the alternative method of the invention can be carried out by deducing the amount of bound malachite green in the second fraction of the sample and by deducing the amount of leucomalachite green and malachite green in the first fraction of the sample, followed by subtracting the amount of leucomalachite green and malachite green in the first fraction of the sample from the amount of bound malachite green in the second fraction of the sample, to yield the amount of leucomalachite green in the sample.

In a further aspect, the invention includes a kit for detecting or determining leucomalachite green and malachite green, the kit including at least one conjugate of the present invention; and at least one antibody of the present invention. The kit may optionally include instructions for the use of said conjugates and said antibodies for detecting leucomalachite green and malachite green. This is a semi-quantitative kit that can, optionally, detect or determine an analyte selected from leucomalachite green, malachite green and mixtures of leucomalachite green and malachite green in a sample. Alternatively, the kit is for determining each of leucomalachite green and malachite green, the kit including at least one conjugate of the present invention, and at least one antibody of the present invention. Such a kit may optionally include instructions for the use of said conjugates and said antibodies for determining the amount of each of leucomalachite green and malachite green in a sample. Preferably, the sample is a solution, such as a biological fluid. More preferably, the sample is serum or urine.

In any individual method and kit of the invention, the basic hapten used to prepare the conjugate and the basic hapten used to prepare the immunogen, against which the antibody is raised, may be the same or different. It is preferred that they be different so as to increase the sensitivity of the assay. Without wishing to be so limited, it is thought that target analytes in a sample are better able to compete for antibody binding sites with a conjugate that is structurally even slightly diverse from the immunogen that raised the antibody. The better sensitivity results so obtained are exemplified in Example 12 herein below. In any individual method and kit of the invention, the method or kit can show a sensitivity or IC₅₀ (ng/ml) of of less than 35 ng/ml, optionally less than 30 ng/ml, each for leucomalachite green. If the basic hapten used to prepare the conjugate and the basic hapten used to prepare the immunogen, against which the antibody is raised, is different, the method or kit can show a sensitivity or IC₅₀ (ng/ml) of less than 10 ng/ml, optionally less than 7.5 ng/ml, further optionally less than 5.0 mg/ml, each for leucomalachite green. In any individual method and kit of the invention, the method or kit can show a sensitivity or IC₅₀ (ng/ml) of less than 150 ng/ml, optionally less than 125 ng/ml, still further optionally less than 105 ng/ml, each for malachite green. If the basic hapten used to prepare the conjugate and the basic hapten used to prepare the immunogen, against which the antibody is raised, is different, the method or kit can show a sensitivity or IC₅₀ (ng/ml) of less than 35 ng/ml, optionally less than 25 ng/ml, further optionally less than 15 ng/ml, each for malachite green.

In a further aspect, the present invention involves use of at least one conjugate according to the present invention, with at least one antibody according to the present invention, to detect or determine leucomalachite green and malachite green in samples such as biological fluids.

### DRAWINGS OF THE INVENTION

An embodiment of the invention is now described by way of example and with reference to the accompanying drawings in which:
FIGURE 1 shows the structural formulae of *p*-(3-carboxypropoxy) leucomalachite green (Hapten A), *p*-(3-carboxypropoxy) malachite green (Hapten B) and p-(3-acetylthiopropanamido)leucomalachite (Hapten C).
FIGURE 2 shows the preparation steps of the hapten *p*-(3-carboxypropoxy) leucomalachite green (Hapten A) of Figure 1.
FIGURE 3 shows the preparation step of the hapten *p*-(3-carboxypropoxy) malachite green (Hapten B) of Figure 1.
FIGURE 4 shows the preparation steps of the hapten p-(3-acetylthiopropanamido) leucomalachite (Hapten C) of Figure 1.
FIGURE 5 shows NMR results for *p*-(3-carboxypropoxy) leucomalachite green (Hapten A) of Figure 1.
FIGURE 6 shows MALDI results for BSA.
FIGURE 7 shows MALDI results for Immunogen I (the haptenp-(3-carboxypropoxy) leucomalachite green (Hapten A) of Figure 1 conjugated to BSA).
FIGURE 8 shows a competitive ELISA microtiter plate assay for the development of competitive ELISAs for leucomalachite green and malachite green.

### DESCRIPTION OF THE INVENTION

### Preparation of Haptens

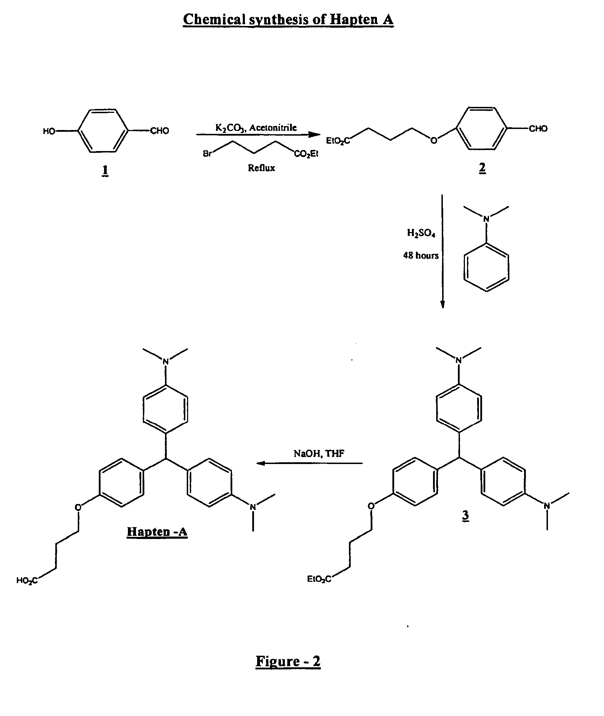

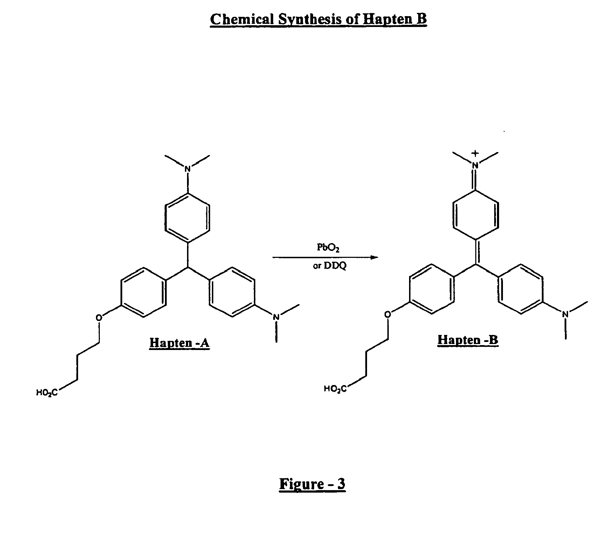

*p*-(3-Carboxypropoxy) leucomalachite green, (hapten **A)** was prepared in three steps according to Figure 2 of the accompanying drawings. Firstly, 4-hydroxy benzaldehyde, **1,** was reacted with ethyl 4-bromobutyrate in the presence of potassium carbonate in acetonitrile at reflux to give *p*-[3-(ethylcarboxy)propoxy] benzaldehyde, **2.** The ester obtained, **2,** was reacted with N, N-dimethylaniline in the presence of sulphuric acid at 120°C for 48 hours to afford *p*-[3-(ethylcarboxy)propoxy)] leucomalachite green, **3,** in moderate yield. The hapten **A** was obtained after saponification of the leucomalachite green ester, **3,** with sodium hydroxide in tetrahydrofuran (THF) and methanol. *p*-(3-Carboxypropoxy) malachite green, hapten **B**, may be obtained by oxidation of hapten **A**, with lead oxide (PbO₂) or 2,3-dichloro-5,6-dicyano-1,4-benzoquinone (DDQ) (Figure 3).

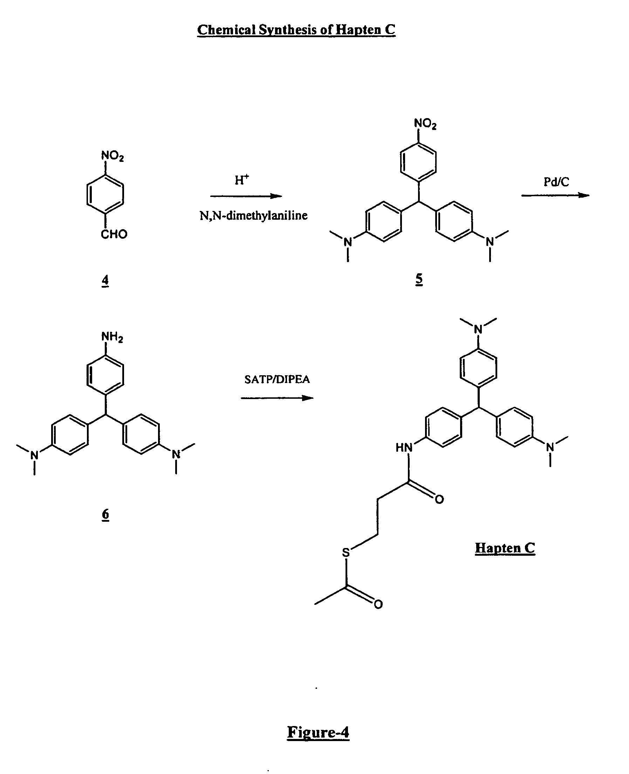

Hapten C, *p*-(3-acetylthiopropanamido)leucomalachite, was prepared in three steps (Figure 4). Firstly, 4-nitrobenzaldehyde, **4,** was heated with N,N-dimethylaniline under acid conditions to give p-nitroleucomalachite, **5.** This was reduced using Pd/C in the presence of ammonium formate in MeOH/THF to give p-aminoleucomalachite, 6. Finally, *p*-aminoleucomalachite was reacted with N-succinimidyl 3-(acetylthio)propionate in the presence of N,N-diisopropylethylamine in 1,4-dioxane and stirred at room temperature overnight to yield hapten C.

### Preparation of Immunogens and Conjugates

Although the haptens disclosed herein provide defined structural epitopes, they are not in themselves immunogenic and therefore need to be conjugated to carrier materials, which will elicit an immunogenic response when administered to a host animal. Appropriate carrier materials commonly contain poly(amino acid) segments and include polypeptides, proteins and glycoproteins. Illustrative examples of useful carrier materials are bovine serum albumin (BSA), egg ovalbumin, bovine gamma globulin, bovine thyroglobulin (BTG), keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. In particular, carbohydrates, yeasts or polysaccharides may be conjugated to the hapten to produce an immunogen.

The haptens disclosed herein can also be coupled to a detectable labelling agent such as an enzyme (for example, horseradish peroxidase), a substance having fluorescent properties or a radioactive label for the preparation of conjugates (or detection reagents) for use in the immunoassays. The fluorescent substance may be, for example, a monovalent residue of fluorescein or a derivative thereof.

Immunogen formation (Examples 7 and 8) involves conventional conjugation chemistry in which the oxygen of the hydroxyl group of Hapten **A** combines first with DCC and then NHS to form an ester with a powerful leaving group. Nucleophilic attack on the carbonyl of the ester functionality by a free amine group on the protein (BSA or BTG), results in an amide bond and formation of the target immunogens. Formation of conjugate Hapten **A**-HRP follows a similar mechanism using EDC and sulfo-NHS (Example 9). Hydrolysis of Hapten **C** to form the thiol derivative, followed by nucleophilic substitution of modified HRP by hydrolysed Hapten **C**, results in the formation of the conjugate (Example 10).

In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS). The immunogens of the present invention are suitable for immunisation, in order to produce antibodies for the detection of leucomalachite green and malachite green.

### General Procedure for MALDI-TOF Analysis of Immunogens.

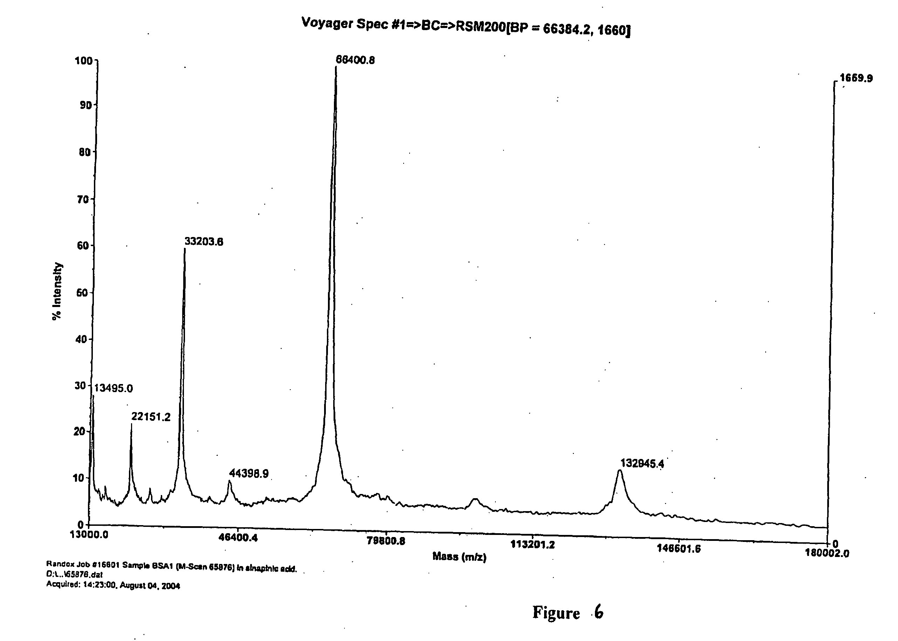

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1µl) were analysed using a matrix of Sinapinic acid and bovine serum albumin (Fluka) was used as an external calibrant. Figure 6 of the accompanying drawings shows the analysis for BSA carrier material. As will be seen, a major signal was present which indicates an average protonated mass for this sample of m/z 66,400. The signal at m/z 33,203 is consistent with the major component in the doubly-charged form. Further signals were observed including m/z 13,495.

### Preparation of Antisera

In order to generate polyclonal antisera, the immunogen of the present invention is mixed with Freund's Adjuvant and the mixture is injected into a host animal, such as rabbit, sheep, mouse, guinea pig or horse. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate nonspecific binding.

The specific antibodies prepared in this invention are useful as reagents in immunoassays for the detection or determination of leucomalachite green and malachite green in biological fluids. The antibodies of the present invention are capable of binding with the metabolite leucomalachite green and with the parent malachite green.

### EXAMPLES

### Example 1: Preparation of p-[3-(Ethylcarboxy) propoxy] benzaldehyde (2).

To a suspension of 4-hydroxy benzaldehyde, 1, (20 g, 164 mMol) and potassium carbonate (68 g, 492 mMol) in acetonitrile (300 ml) was added ethyl 4-bromobutyrate (35.2 ml, 246 mMol). The mixture was then heated at reflux overnight. After cooling to room temperature, the solid was filtered off and the solvent was removed *in vacuo.* Water (200 ml) was then added to the residue and extracted with ethyl acetate (3 x 200 ml). The combined organic phases were washed with brine (1 x 200 ml), dried over sodium sulfate, filtered and evaporated to dryness. The residue so obtained was purified by flash chromatography on silica gel using 30% ethyl acetate in hexane as eluant to give **2** as a colourless oil (27 g, 69.8%).
IR spectrum (film): 1733.7, 1692.5, 1601.2, 1577.9, 1509.8, 1257.7, 1161.1cm⁻¹.

### Example 2: Preparation of p-[3-(Ethylcarboxy)propoxy]leucomalachite green (3).

To a mixture of p-[3-(ethylcarboxy)propoxy]benzaldehyde **2** (10 g, 42.4 mMol) and N,N-dimethylaniline (12.38 g, 106 mMol) was added concentrated sulphuric acid (2 ml) and the mixture was heated at 120°C for 48 hours. The solution was then cooled to room temperature, water (200 ml) was added and the mixture neutralised with solid sodium carbonate before extracting with ethyl acetate (3 x 100 ml), washed with brine (1 x 100 ml) dried over sodium sulfate, filtered and concentrated *in vacuo* to dryness. The crude product obtained was purified by chromatography on silica gel using 25% ethyl acetate in hexane as eluant to give the ester, **3**, as an orange oil (6.9 g, 35%).
IR spectrum (film): 1735.3, 1612.2, 1518.3, 1242.6cm⁻¹.

### Example 3: Preparation of p-(3-Carboxypropoxy)leucomalachite green (Hapten A)

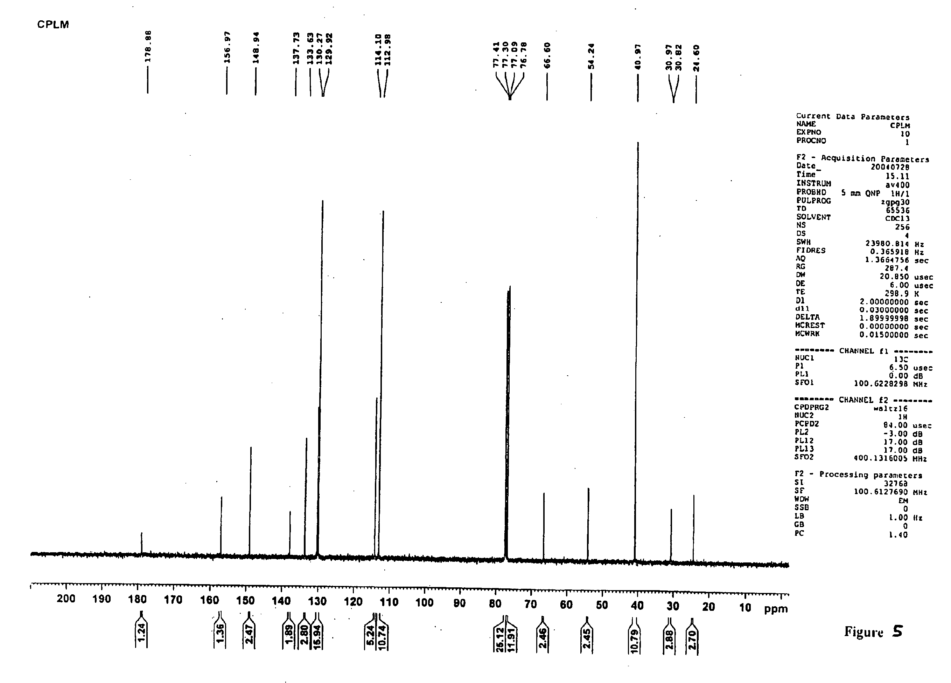

To a solution of the ester, **3**, (8.4 g, 18.75 mMol) in a mixture of methanol and THF (2:1, 240 ml) was added sodium hydroxide (2N, 40 ml). The mixture was stirred at room temperature for 16 hours. The solvents were removed *in vacuo* and water (100 ml) was added. The solution was neutralised to pH7 by addition of HCl (2N) and the resulting precipitate obtained was collected by filtration and dried in a dessicator over phosphorus pentoxide. The hapten **A** was obtained as a blue/green solid (3.2 g, 40%).
NMR ¹³C, solvent δ-MeOH (Figure 5): 178.8, 156.9, 148.9, 137.7, 133.6, 130.6, 129.9, 114.1, 112.9, 66.6, 54.2, 40.9, 30.9, 24.6.

### Example 4: Preparation of p-Nitroleucomalachite

To *p*-nitrobenzaldehyde, **4**, (12.81 g, 84.77 mMol) and N,N-dimethylaniline (24.76 g, 204.3 mMol) was added sulphuric acid (5 ml). After heating for 48 hours, water (200 ml) was added and the solution neutralised by the addition of saturated sodium carbonate solution. The solution was extracted with dichloromethane (3 x 200 ml) and ethyl acetate (3 x 200 ml), dried over sodium sulphate, filtered and evaporated to dryness. The crude product was purified by column chromatography using (silica gel; 20% ethyl acetate in hexane), to give p-nitroleucomalachite, **5,** as a yellow solid (3.5 g, 11 %).
NMR ¹³C, solvent CDCl₃: 153.5, 149.5, 146.2, 131.0, 129.4, 123.4, 113.9, 54.9, 40.9

### Example 5: Preparation of p-Aminoleucomalachite

To *p*-nitroleucomalachite (2.1 g, 5.6 mMol) in a mixture of methanol and tetrahydrofuran (1:1, 160 ml)) was added ammonium formate (1.91 g, 30.29 mMol) and Pd/C (300 mg, 2.83 mMol) and the mixture stirred at room temperature for 3 hours. Solvent was removed *in vacuo,* water (200 ml) added, and the resultant solution extracted with ethyl acetate (3 x 200 ml), dried over sodium sulphate and evaporated to dryness. The crude product was purified by column chromatography using (silica gel; 10% hexane in ethyl acetate), to give *p-*aminoleucomalachite, **6,** as a waxy purple/red solid (2.43 g, 75%).

### Example 6: Preparation of p-(3-Acetylthiopropanamido)leucomalachite (Hapten C)

*p*-Aminoleucomalachite (470 mg, 1.36 mMol), N,N-diisopropylethylamine (350 µl, 2.04 mMol) and N-succinimidyl 3-(acetylthio)propionate (367 mg, 1.5 mMol) were stirred in 1,4-dioxane (25 ml) at room temperature overnight. The solvent was removed *in vacuo.* The crude product was purified by column chromatography (silica gel; ethyl acetate:hexane, 1:5) to give p-(3-acetylthiopropanamido) leucomalachite, hapten **C,** as a purple oil (560 mg, 87%).
IR spectrum (film): 1741, 1692, 1611, 1518, 1353, 1204 cm⁻¹

### Example 7: Conjugation of Hapten A to BSA (Preparation of Immunogen I)

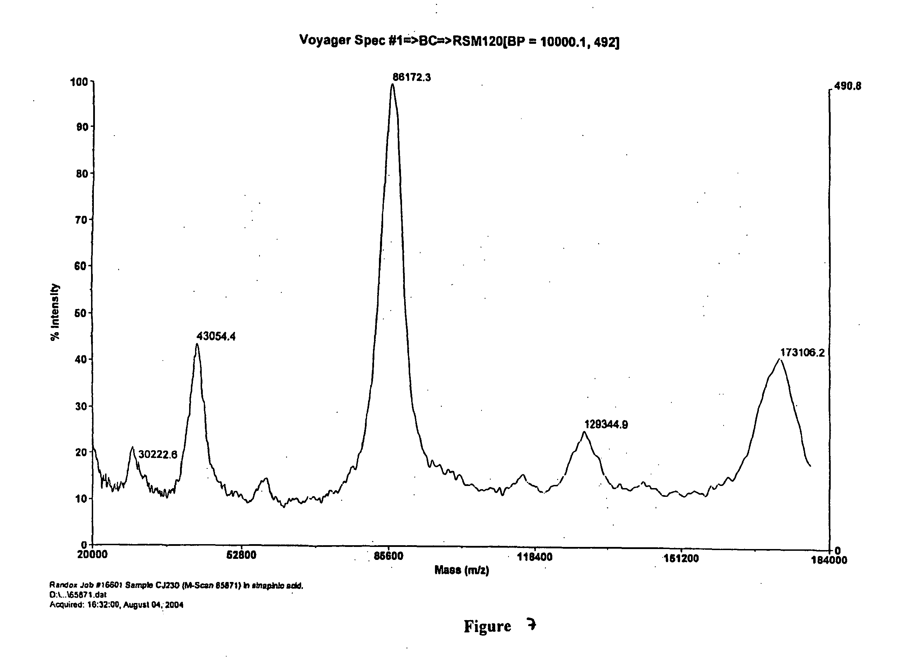

To a solution of hapten **A** (60 mg, 0.14 mMol) in DMF (2 ml) was added N-hydroxy succinimide (17.7 mg, 0.154 mMol) and N,N-dicyclohexylcarbodiimide (DCC) (31.8 mg, 0.154 mMol) and the mixture stirred at room temperature overnight. The dicyclohexylurea formed was removed by filtration and the filtrate added dropwise to a solution of BSA (200 mg) in 0.1M sodium bicarbonate (pH 8.5, 9 ml). The mixture was stirred at room temperature overnight. The solution was dialysed against 50 mM phosphate buffer, pH 7.2 (3 changes) for 24 hours at 4°C and then freeze-dried.
MALDI results showed 47.9 molecules of hapten **A** had been conjugated to 1 molecule of BSA (Figure 7).

### Example 8: Conjugation of Hapten A to BTG (Preparation of Immunogen II)

The conjugation of hapten **A** to BTG was carried out by the same method as Example 7 by using: Hapten **A** (80 mg), N-hydroxysuccinimide (24 mg), DCC (36 mg) and BTG (150 mg).

### Example 9: Conjugation of Hapten A to HRP (horseradish peroxidase).

EDC.HCl (10 mg) was dissolved in water (0.5 ml) and immediately added to a solution of hapten **A** (2mg) in DMF (0.2 ml). After mixing this solution was added dropwise to a solution of HRP (20 mg) in water (1 ml). Sulfo-NHS (5 mg) was added and the reaction mixture was incubated in the dark at room temperature overnight. Excess hapten was removed by desalting with PD-10 columns (Pharmacia) in series, pre-equilibrated with PBS (phosphate buffered saline) at pH 7.2. The hapten-HRP conjugate was then dialysed overnight against 10 L of PBS/pH 7.2 at 4°C.

### Example 10: Conjugation of p-(3-Acetylthiopropanamido)leucomalachite to HRP (horseradish peroxidase)

A solution of 1 M potassium hydroxide (100 ml) was added to a solution to *p*-(3-acetylthiopropanamido)leucomalachite (2 mg) in DMF (0.2 ml). After incubating for 10 minutes, 0.1 M phosphate buffer was added, followed by 1 M HCl. After mixing, this solution was added dropwise to a solution of modified HRP (20 mg). Excess hapten was removed by desalting with PD-10 columns (Pharmacia) in series, pre-equilibrated with PBS (phosphate buffered saline) at pH 7.2. The hapten-HRP conjugate was then dialysed overnight against 10 L of PBS/ pH 7.2 at 4°C.

### Example 11: Preparation of antibodies to Immunogen II, prepared in Example 8.

An aqueous solution of immunogen **II** was formulated with Freund's Complete Adjuvant (FCA) to form an emulsion consisting of 2 mg/ml immunogen in 50% (v/v) FCA. Three sheep were immunised with this emulsion (1° immunisation), 0.25 ml being intramuscularly injected at each of four sites in the rump of each animal. Subsequent immunizations (boosts) contained 1mg/ml immunogen. All boosts were emulsified in 50% (v/v) Freund's Incomplete Adjuvant (FIA) and were administered in the same manner as the 1 immunisation, at monthly intervals for 1 year. Blood sampling took place 7 to 14 days after each boost. Each sample was processed to produce antiserum, which was further purified by caprylic acid and ammonium sulfate precipitation to yield an immunoglobulin (Ig) fraction. The Ig fraction was evaluated by competitive ELISA microtiter plate assay, as described in Example 12 below.

### Example 12: Development of ELISAs for Leucomalachite green and Malachite green.

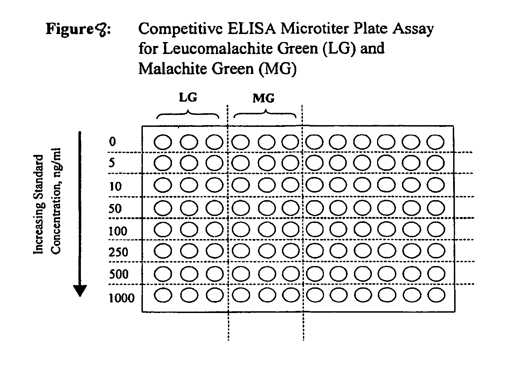

The wells of an enhanced binding 96 well polystyrene microtiter plate were coated with the Ig fraction of the antiserum raised to immunogen **II** (hapten **A**-BTG) (Example 8), diluted in 10 mM Tris, pH8.5 (125 µl/well). The appropriate antibody coating dilution was determined using standard ELISA checkerboard techniques. The plate was incubated for 2 hours at 37°C, washed 4 times with Tris buffered saline containing Tween 20 (TBST) and tapped dry. Standard solutions of leucomalachite green and malachite green were prepared in TBST at 0, 5, 10, 50, 100, 250, 500 and 1000 ng/ml, and 50 µl of each was added to the appropriate wells (Figure 8).

75 µl of conjugate I (hapten A-HRP) (Example 9), diluted in Tris buffer (pH 7.2) containing EDTA, D-mannitol, sucrose, thimerosal and BSA, was added to each of the wells, as shown in Figure 8. The appropriate dilution of conjugate was also determined using standard ELISA checkerboard techniques. The plate was incubated at 37°C for 2 hours. Excess unbound conjugate was removed by washing 6 times over a 10 minute period with TBST. 125 µl of tetramethylbenzidine (TMB) substrate solution was added to each well of the plate that was then incubated for 15 to 20 minutes in the dark at room temperature. The reaction was terminated by addition of 125 µl 0.2M H₂SO₄ to each well. The absorbance was then measured at 450 nm using a microtiter plate reader. The data generated in the assay is presented in Table 1.

**Table 1: Data generated from competitive microtiter plate assay for leucomalachite green and malachite green, employing antisera generated to immunogen II (hapten A-BTG) (Example 8 and Conjugate I (Example 9)).**

| **Standard Concentration** | **Leucomalachite Green** | | **Malachite Green** | |
|---|---|---|---|---|
| **ng/ml** | **A₄₅₀** | **%B/B₀** | **A₄₅₀** | **%B/B₀** |
| 0 | 1.814 | 100 | 1.691 | 100 |
| 5 | 1.378 | 76.0 | 1.440 | 85.2 |
| 10 | 1.225 | 67.5 | 1.392 | 82.3 |
| 50 | 0.762 | 42.0 | 1.075 | 63.6 |
| 100 | 0.547 | 30.2 | 0.869 | 51.4 |
| 250 | 0.366 | 20.2 | 0.664 | 39.3 |
| 500 | 0.247 | 13.6 | 0.520 | 30.8 |
| 1000 | 0.178 | 9.8 | 0.416 | 24.6 |
| | | | | |
| **IC₅₀ (ng/ml)** | 27.33 | | 103.39 | |
| **%CR** | 100 | | 26.43 | |

| | | | | |
|---|---|---|---|---|
| *A₄₅₀* = absorbance at 450 nm B = absorbance at 450 nm at xng/ml standard concentration B₀ = absorbance at 450 nm at 0ng/ml standard concentration IC₅₀ = standard concentration which produces 50% B/B₀ %CR = percentage cross-reactivity based on specificity to leucomalachite green - this is what is meant by the term "cross-reactivity" in the present specification. | | | | |

75 µl of conjugate C (hapten C-HRP) (Example 10), diluted in Tris buffer (pH 7.2) containing EDTA, D-mannitol, sucrose, thimerosal and BSA, was added to each of the wells, as shown in Figure 8. The appropriate dilution of conjugate was also determined using standard ELISA checkerboard techniques. The plate was incubated at 25°C for 1 hour. Excess unbound conjugate was removed by washing 6 times over a 10-15 minute period with TBST.

125 µl of tetramethylbenzidine (TMB) substrate solution was added to each well of the plate that was then incubated for 20 minutes in the dark at room temperature. The reaction was terminated by addition of 100 ul 0.2 M H₂SO₄ to each well. The absorbance was then measured at 450 nm using a microtiter plate reader. The data generated in the assay is presented in Table 2.

**Table 2: Data generated from competitive microtiter plate assay for leucomalachite green and malachite green, employing antisera generated to immunogen II (hapten A-BTG) (Example 8) and Conjugate II (Example 10).**

| **Standard Concentration** | **Leucomalachite Green** | | **Malachite Green** | |
|---|---|---|---|---|
| **ng/ml** | **A₄₅₀** | **%B/B₀** | **A₄₅₀** | **%B/B₀** |
| 0 | 1.725 | 100 | 1.692 | 100 |
| 0.5 | 1.550 | 90 | 1.611 | 95 |
| 1 | 1.323 | 77 | 1.504 | 89 |
| 5 | 0.772 | 45 | 1.137 | 67 |
| 10 | 0.538 | 31 | 0.967 | 57 |
| 50 | 0.184 | 11 | 0.497 | 29 |
| 100 | n/a | n/a | 0.342 | 20 |
| 250 | n/a | n/a | 0.227 | 13 |
| 500 | n/a | n/a | 0.184 | 11 |
| 1000 | n/a | n/a | 0.113 | 7 |
| | | | | |
| **IC₅₀ (ng/ml)** | 3.74 | | 13.962 | |
| **%CR** | 100 | | 26.8 | |

| | | | | |
|---|---|---|---|---|
| *A₄₅₀ =* absorbance at 450 nm B = absorbance at 450 nm at xng/ml standard concentration B₀ = absorbance at 450 nm at 0ng/ml standard concentration IC₅₀ = standard concentration which produces 50% B/B₀ %CR = percentage cross-reactivity based on specificity to leucomalachite green. | | | | |

Table 2 shows a sensitivity or IC₅₀ (ng/ml) of 3.74, whilst Table 1 shows a sensitivity or IC₅₀ (ng/ml) of 27.33, each for leucomalachite green. Table 2 shows a sensitivity or IC₅₀ (ng/ml) of 13.962, whilst Table 1 shows a sensitivity or IC₅₀ (ng/ml) of 103.39, each for malachite green. These sensitivity differences have arisen, it is thought, by utilising a conjugate with a crosslinking group that is structurally diverse from the crosslinking group of the immunogen. Without wishing to be bound by theory, it is thought that the analytes in the assay are better able to compete with the conjugate for antibody binding sites, thereby increasing the assay sensitivity while retaining its specificity profile.

## Claims

1. An immunogen comprising leucomalachite green, coupled by way of a crosslinker, to an antigenicity-conferring carrier material, the crosslinker extending from the para, ortho or meta-position of the unsubstituted phenyl ring of leucomalachite green.

2. An immunogen according to Claim 1, in which the crosslinker comprises -X-Y-Z: 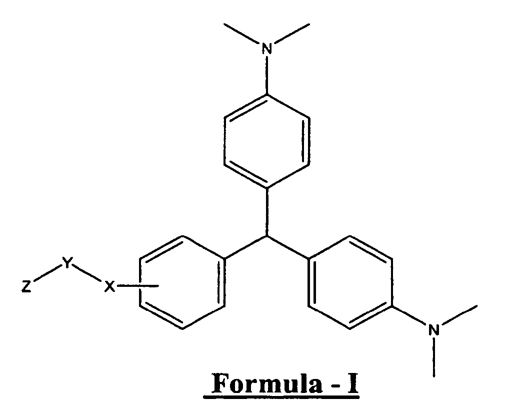 , in which X is a first bivalent link, Y is a second bivalent link and Z is a reactive group capable of coupling to the antigenicity-conferring carrier material.

3. An immunogen as claimed in claim 1 or 2, in which the crosslinker extends from the para-position of the unsubstituted phenyl ring of leucomalachite green.

4. An immunogen as claimed in Claim 2 or 3, in which X is a heteroatom selected from O, S and N, in which X is most preferably an O atom.

5. An immunogen as claimed in any one of Claims 2 to 4, in which Y is a C₁₋₁₀, more preferably a C₂₋₆, most preferably a C₃, substituted or unsubstituted straight chain, saturated alkylene moiety, or an arylene moiety.

6. An immunogen as claimed in any one of Claims 2 to 5, in which Z (before conjugation with the antigenicity-conferring carrier material) is selected from a carboxy, a dithiopyridyl, a maleimide, an amino, a hydroxyl, a thiol, a thioester or an aldehyde moiety, in which Z (before conjugation with the antigenicity-conferring carrier material) is, optionally, a carboxy (COOH) moiety.

7. An immunogen comprising *p*-(3-carboxypropoxy) leucomalachite green coupled to an antigenicity-conferring carrier material.

8. The immunogen of any one of Claims 1 to 7, in which the carrier material is selected from a protein, a protein fragment, a synthetic polypeptide or a semisynthetic polypeptide.

9. An antibody raised against the immunogen of any one of Claims 1 to 8, the antibody being capable of binding with at least one structural epitope of leucomalachite green and malachite green.

10. An antibody raised against the immunogen of any one of Claims 1 to 8, the antibody having specificity for leucomalachite green **characterised by** having cross-reactivity for malachite green, in which the antibodies optionally have cross-reactivity of more than 10% for malachite green.

11. A conjugate comprising leucomalachite green, covalently bonded, by way of a crosslinker, to a detectable labelling agent, the crosslinker extending from the para, ortho or meta-position of the unsubstituted phenyl ring of leucomalachite green.

12. The conjugate according to Claim 11, in which the crosslinker comprises -X-Y-Z: 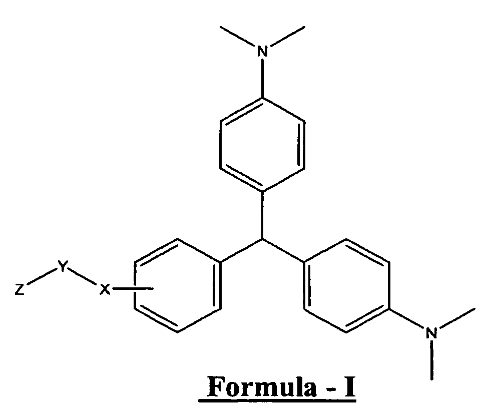 , in which X is a first bivalent link, Y is a second bivalent link and Z is a reactive group capable of coupling to the detectable labelling agent.

13. The conjugate as claimed in Claim 11 or 12, in which the crosslinker extends from the para-position of the unsubstituted phenyl ring of leucomalachite green.

14. The conjugate as claimed in Claim 12 or 13, in which X is a heteroatom selected from O, S and N, in which X is most preferably an N atom.

15. The conjugate as claimed in any one of Claims 12 to 14, in which Y is a C₁₋₁₀, more preferably a C₂₋₆, most preferably a C₃, substituted or unsubstituted straight chain, saturated alkylene moiety, or an arylene moiety.

16. The conjugate as claimed in any one of Claims 12 to 15, in which Z (before conjugation with the detectable labelling agent) is selected from a carboxy, a dithiopyridyl, a maleimide, an amino, a hydroxyl, a thiol, a thioester or an aldehyde moiety.

17. The conjugate as claimed in any one of Claims 12 to 16, in which Z (before conjugation with the detectable labelling agent) is a thiol or a thioester.

18. A conjugate comprising *p*-(3-thiopropanamido) leucomalachite coupled to a detectable labelling agent.

19. A conjugate according to any one of Claims 11 to 18, in which the detectable labelling agent is selected from an enzyme, a luminescent substance and a radioactive substance.

20. A method for detecting or determining leucomalachite green and malachite green in a sample, the method comprising contacting the sample with at least one conjugate of any one of Claims 11 to 19 and with at least one antibody of Claim 9 or 10; detecting bound conjugate; and deducing from a calibration curve the presence of, or the amount of, leucomalachite green and malachite green in the sample.

21. A method for determining the amount of each analyte in a sample, in which the analytes comprise leucomalachite green and malachite green, the method comprising contacting the sample with at least one conjugate of any one of Claims 11 to 19, and with at least one antibody of Claim 9 or 10 and determining bound conjugate; contacting the sample with at least one antibody of Claim 9 or 10 and determining bound malachite green; and deducing from calibration curves, the amount of each of leucomalachite green and malachite green in the sample.

22. A kit for detecting or determining leucomalachite green and malachite green, the kit including at least one conjugate of any one of Claims 11 to 19; and at least one antibody of Claim 9 or 10.

23. Use of at least one conjugate of any one of Claims 11 to 19 with at least one antibody of Claim 9 or 10 to detect or determine leucomalachite green and malachite green in samples such as biological fluids.

## Patentansprüche

1. Immunogen, das Leukomalachitgrün umfasst, das durch einen Crosslinker mit einem Antigenität übertragenden Trägermaterial gekuppelt ist, wobei sich der Crosslinker von der para-, ortho- oder meta-Position des unsubstituierten Phenylrings von Leukomalachitgrün erstreckt.

2. Immunogen nach Anspruch 1, worin der Crosslinker -X-Y-Z umfasst: 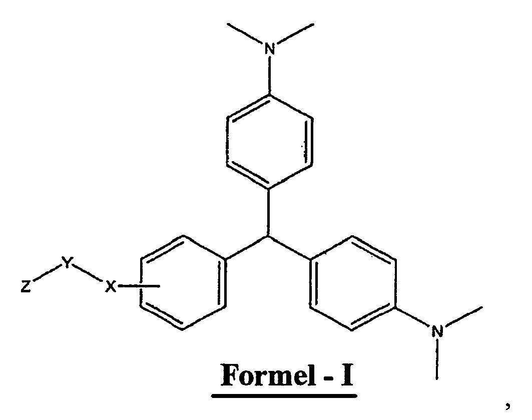 worin X ein erstes bivalentes Verbindungsglied ist, Y ein zweites bivalentes Verbindungsglied ist und Z eine reaktive Gruppe ist, die zum Antigenität übertragenden Trägermaterial kuppeln kann.

3. Immunogen nach Anspruch 1 oder 2, worin sich der Crosslinker von der para-Position des unsubstituierten Phenylrings von Leukomalachitgrün erstreckt.

4. Immunogen nach Anspruch 2 oder 3, worin X ein aus O, S und N ausgewähltes Heteroatom ist, worin X am bevorzugtesten ein O-Atom ist.

5. Immunogen nach einem der Ansprüche 2 bis 4, worin Y eine C₁₋₁₀-, bevorzugter eine C₂₋₆-, am bevorzugtesten eine C₃-, substituierte oder unsubstituierte geradkettige, gesättigte Alkyleneinheit oder eine Aryleneinheit ist.

6. Immunogen nach einem der Ansprüche 2 bis 5, worin Z (vor der Konjugation mit dem Antigenität übertragenden Trägermaterial) aus einer Carboxy-, einer Dithiopyridyl-, einer Maleinimid-, einer Amino-, einer Hydroxyl-, einer Thiol-, einer Thioester- oder einer Aldehydeinheit ausgewählt ist, worin Z (vor der Konjugation mit dem Antigenität übertragenden Trägermaterial) optional eine Carboxy(COOH)-Einheit ist.

7. Immunogen, das *p*-(3-Carboxypropoxy)-Leukomalachitgrün umfasst, das zu einem Antigenität übertragenden Trägermaterial gekuppelt ist.

8. Immunogen nach einem der Ansprüche 1 bis 7, worin das Trägermaterial aus einem Protein, einem Proteinfragment, einem synthetischen Polypeptid oder einem halbsynthetischen Polypeptid ausgewählt ist.

9. Antikörper, der gegen das Immunogen nach einem der Ansprüche 1 bis 8 produziert wurde, wobei der Antikörper zu mindestens einem strukturellen Epitop von Leukomalachitgrün und Malachitgrün binden kann.

10. Antikörper, der gegen das Immunogen nach einem der Ansprüche 1 bis 8 produziert wurde, wobei der Antikörper eine Spezifität für Leukomalachitgrün aufweist, die durch Kreuzreaktivität für Malachitgrün **gekennzeichnet** ist, worin die Antikörper optional eine Kreuzreaktivität von mehr als 10 % für Malachitgrün aufweisen.

11. Konjugat, das Leukomalachitgrün umfasst, das kovalent durch einen Crosslinker zu einem nachweisbaren Markierungsagens gebunden ist, wobei sich der Crosslinker von der para-, ortho- oder meta-Position des unsubstituierten Phenylrings von Leukomalachitgrün erstreckt.

12. Konjugat nach Anspruch 11, worin der Crosslinker -X-Y-Z umfasst: 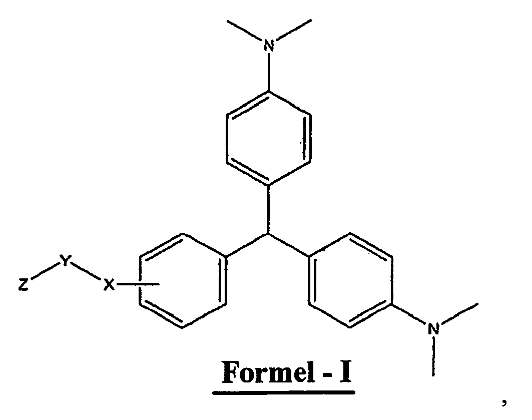 worin X ein erstes bivalentes Verbindungsglied ist, Y ein zweites bivalentes Verbindungsglied ist und Z eine reaktive Gruppe ist, die zum nachweisbaren Markierungsagens kuppeln kann.

13. Konjugat nach Anspruch 11 oder 12, worin sich der Crosslinker von der para-Position des unsubstituierten Phenylrings von Leukomalachitgrün erstreckt.

14. Konjugat nach Anspruch 12 oder 13, worin X ein aus O, S und N ausgewähltes Heteroatom ist, worin X am bevorzugtesten ein N-Atom ist.

15. Konjugat nach einem der Ansprüche 12 bis 14, worin Y eine C₁₋₁₀-, bevorzugter eine C₂₋₆-, am bevorzugtesten eine C₃-, substituierte oder unsubstituierte geradkettige, gesättigte Alkyleneinheit oder eine Aryleneinheit ist.

16. Konjugat nach einem der Ansprüche 12 bis 15, worin Z (vor der Konjugation mit dem nachweisbaren Markierungsagens) aus einer Carboxy-, einer Dithiopyridyl-, einer Maleinimid-, einer Amino-, einer Hydroxyl-, einer Thiol-, einer Thioester- oder einer Aldehydeinheit ausgewählt ist.

17. Konjugat nach einem der Ansprüche 12 bis 16, worin Z (vor der Konjugation mit dem nachweisbaren Markierungsagens) ein Thiol oder ein Thioester ist.

18. Konjugat, das *p*-(3-Thiopropanamido)-Leukomalachit umfasst, das zu einem nachweisbaren Markierungsagens gekuppelt ist.

19. Konjugat nach einem der Ansprüche 11 bis 18, worin das nachweisbare Markierungsagens aus einem Enzym, einer lumineszierenden Substanz und einer radioaktiven Substanz ausgewählt ist.

20. Verfahren zum Nachweis oder zur Bestimmung von Leukomalachitgrün und Malachitgrün in einer Probe, wobei das Verfahren Folgendes umfasst: Inkontaktbringen der Probe mit mindestens einem Konjugat nach einem der Ansprüche 11 bis 19 und mit mindestens einem Antikörper nach Anspruch 9 oder 10; Nachweis von gebundenem Konjugat; und Ableitung der Gegenwart von oder der Menge von Leukomalachitgrün und Malachitgrün in der Probe aus einer Kalibrierungskurve.

21. Verfahren zur Bestimmung der Menge von jedem Analyten in einer Probe, worin die Analyten Leukomalachitgrün und Malachitgrün umfassen, wobei das Verfahren Folgendes umfasst: Inkontaktbringen der Probe mit mindestens einem Konjugat nach einem der Ansprüche 11 bis 19 und mit mindestens einem Antikörper nach Anspruch 9 oder 10 und Bestimmung von gebundenem Konjugat; Inkontaktbringen der Probe mit mindestens einem Antikörper nach Anspruch 9 oder 10 und Bestimmung von gebundenem Malachitgrün; und Ableitung der Menge von jeweils Leukomalachitgrün und Malachitgrün in der Probe aus Kalibrierungskurven.

22. Kit zum Nachweis oder zur Bestimmung von Leukomalachitgrün und Malachitgrün, wobei der Kit mindestens ein Konjugat nach einem der Ansprüche 11 bis 19 und mindestens einen Antikörper nach Anspruch 9 oder 10 einschließt.

23. Verwendung von mindestens einem Konjugat nach einem der Ansprüche 11 bis 19 mit mindestens einem Antikörper nach Anspruch 9 oder 10 zum Nachweis oder zur Bestimmung von Leukomalachitgrün und Malachitgrün in Proben, wie zum Beispiel biologischen Flüssigkeiten.

## Revendications

1. Immunogène comprenant du vert leucomalachite, couplé au moyen d'un agent de réticulation à une substance support conférant une antigénicité, l'agent de réticulation s'étendant de la position para, ortho ou méta du cycle phényle non substitué du vert leucomalachite.

2. Immunogène selon la revendication 1, dans lequel l'agent de réticulation comprend -X-Y-Z: 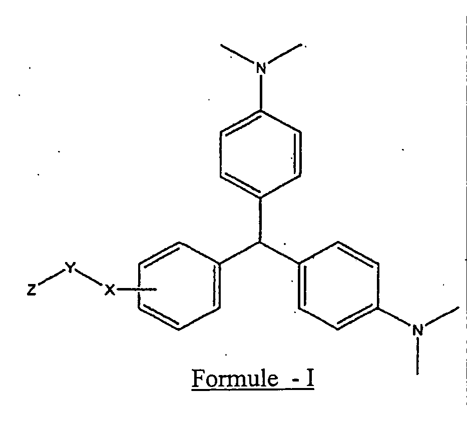 dans laquelle X est une première liaison bivalente, Y est une deuxième liaison bivalente et Z est un groupe réactif capable de se coupler à la substance support conférant une antigénicité.

3. Immunogène selon la revendication 1 ou 2, dans lequel l'agent de réticulation s'étend de la position para du cycle phényle non substitué du vert leucomalachite.

4. Immunogène selon la revendication 2 ou 3, dans lequel X est un hétéroatome choisi parmi O, S et N, dans lequel X est, le plus préférablement, un atome de O.

5. Immunogène selon l'une quelconque des revendications 2 à 4, dans lequel Y est un motif alkylène en C₁₋₁₀, plus préférablement en C₂₋₆, le plus préférablement en C₃, saturé, à chaîne linéaire, substitué ou non substitué, ou un motif arylène.

6. Immunogène selon l'une quelconque des revendications 2 à 5, dans lequel Z (avant la conjugaison avec la substance support conférant une antigénicité) est choisi parmi un motif carboxy, dithiopyridyle, maléimide, amino, hydroxyle, thiol, thioester ou aldéhyde, dans lequel Z (avant la conjugaison avec la substance support conférant une antigénicité) est, éventuellement, un motif carboxy (COOH).

7. Immunogène comprenant du vert leucomalachitep-(3-carboxypropoxy) couplé à une substance support conférant une antigénicité.

8. Immunogène selon l'une quelconque des revendications 1 à 7, dans lequel la substance support est choisie parmi une protéine, un fragment protéique, un polypeptide synthétique ou un polypeptide semi-synthétique.

9. Anticorps dirigé contre l'immunogène de l'une quelconque des revendications 1 à 8, l'anticorps étant capable de se lier à au moins un épitope structural de vert leucomalachite et de vert malachite.

10. Anticorps dirigé contre l'immunogène de l'une quelconque des revendications 1 à 8, l'anticorps présentant une spécificité pour le vert leucomalachite, **caractérisé par le fait qu'**il présente une réactivité croisée pour le vert malachite, dans lequel les anticorps présentent éventuellement une réactivité croisée de plus de 10 % pour le vert malachite.

11. Conjugué comprenant du vert leucomalachite lié de manière covalente au moyen d'un agent de réticulation à un agent de marquage détectable, l'agent de réticulation s'étendant de la position para, ortho ou méta du cycle phényle non substitué du vert leucomalachite.

12. Conjugué selon la revendication 11, dans lequel l'agent de réticulation comprend -X-Y-Z : 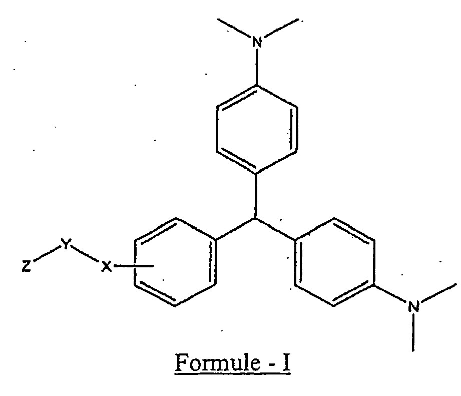 dans laquelle X est une première liaison bivalente, Y est une deuxième liaison bivalente et Z est un groupe réactif capable de se coupler à l'agent de marquage détectable.

13. Conjugué selon la revendication 11 ou 12, dans lequel l'agent de réticulation s'étend de la position para du cycle phényle non substitué du vert leucomalachite.

14. Conjugué selon la revendication 12 ou 13, dans lequel X est un hétéroatome choisi parmi O, S et N, dans lequel X est, le plus préférablement, un atome de N.

15. Conjugué selon l'une quelconque des revendications 12 à 14, dans lequel Y est un motif alkylène en C₁₋₁₀, plus préférablement en C₂₋₆, le plus préférablement en C₃, saturé, à chaîne linéaire, substitué ou non substitué, ou un motif arylène.

16. Conjugué selon l'une quelconque des revendications 12 à 15, dans lequel Z (avant la conjugaison avec l'agent de marquage détectable) est choisi parmi un motif carboxy, dithiopyridyle, maléimide, amino, hydroxyle, thiol, thioester ou aldéhyde.

17. Conjugué selon l'une quelconque des revendications 12 à 16, dans lequel Z (avant la conjugaison avec l'agent de marquage détectable) est un thiol ou un thioester.

18. Conjugué comprenant du leucomalachitep-(3-thiopropanamido) couplé à un agent de marquage détectable.

19. Conjugué selon l'une quelconque des revendications 11 à 18, dans lequel l'agent de marquage détectable est choisi parmi une enzyme, une substance luminescente et une substance radioactive.

20. Méthode de détection ou de détermination du vert leucomalachite et du vert malachite dans un échantillon, la méthode comprenant la mise en contact de l'échantillon avec au moins un conjugué selon l'une quelconque des revendications 11 à 19 et avec au moins un anticorps selon la revendication 9 ou 10 ; la détection de conjugué lié ; et la déduction à partir d'une courbe d'étalonnage de la présence ou de la quantité de vert leucomalachite et de vert malachite dans l'échantillon.

21. Méthode de détermination de la quantité de chaque analyte dans un échantillon, dans laquelle les analytes comprennent le vert leucomalachite et le vert malachite, la méthode comprenant la mise en contact de l'échantillon avec au moins un conjugué selon l'une quelconque des revendications 11 à 19 et avec au moins un anticorps selon la revendication 9 ou 10 et la détermination de conjugué lié ; la mise en contact de l'échantillon avec au moins un anticorps selon la revendication 9 ou 10 et la détermination de vert malachite lié ; et la déduction à partir de courbes d'étalonnage de la quantité de chacun parmi le vert leucomalachite et le vert malachite dans l'échantillon.

22. Kit de détection ou de détermination de vert leucomalachite et de vert malachite, le kit comportant au moins un conjugué selon l'une quelconque des revendications 11 à 19 ; et au moins un anticorps selon la revendication 9 ou 10.

23. Utilisation d'au moins un conjugué selon l'une quelconque des revendications 11 à 19 avec au moins un anticorps selon la revendication 9 ou 10 pour détecter ou déterminer le vert leucomalachite et le vert malachite dans des échantillons tels que des fluides biologiques.
